# EUROPÄISCHE PATENTSCHRIFT

(11) **EP 1 083 984 B1**
(45) Veröffentlichungstag und Bekanntmachung des Hinweises auf die Patenterteilung: **08.03.2006**
(21) Anmeldenummer: 00931008.7
(22) Anmeldetag: 07.04.2000
(51) Int. Cl.: B01F 9/00, A61F 2/06, C12M 3/04

(54) **VERFAHREN ZUR MEHRSCHICHTIGEN BESIEDLUNG VON SUBSTRATEN MIT BIOLOGISCHEN ZELLEN UND DAFUR VERWENDBARE BESIEDLUNGSVORRICHTUNGEN**
METHOD FOR MULTILAYERED POPULATING SUBSTRATES WITH BIOLOGICAL CELLS AND POPULATING DEVICES THAT CAN BE USED THEREFOR
TECHNIQUE PERMETTANT D'ENSEMENCER DES SUBSTRATS A PLUSIEURS COUCHES AVEC DES CELLULES BIOLOGIQUES ET DISPOSITIFS D'ENSEMENCEMENT ASSOCIES

(30) Priorität: 07.04.1999 DE 19915610
(43) Veröffentlichungstag der Anmeldung: 21.03.2001
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen
(86) Internationale Anmeldenummer: PCT/DE2000/001047
(87) Internationale Veröffentlichungsnummer: WO 2000/059618

(56) Entgegenhaltungen:
- EP-A- 0 320 441
- EP-A- 0 704 244
- WO-A-92/22634
- WO-A-93/01843
- WO-A-97/49799
- WO-A-98/32367
- GB-A- 2 002 814
- US-A- 5 035 708
- US-A- 5 155 035
- US-A- 5 492 826
- US-A- 6 010 573

## Beschreibung

Die Erfindung betrifft ein Verfahren zur mehrschichtigen Besiedlung von Substraten mit lebenden biologischen Zellen und dafür verwendbare Besiedlungsvorrichtungen.

Es ist für verschiedene Zwecke erwünscht, Substrate bzw. Matrizes mit Zellen zu besiedeln, die dann auf diesen Substraten aufwachsen sollen. Als Substrate kommen sowohl solche natürlichen Ursprungs als auch synthetische Substrate in Frage. Beispiele für Substrate natürlichen Ursprungs können u.a. Collagensubstrate sein. Synthetische Substrate können beispielsweise aus biologisch inerten Kunststoffen bestehen. Die mit lebenden Zellen zu besiedelnden Substrate haben häufig eine netz- oder schwammartige Struktur, um das Einwachsen der Zellen zu erleichtern.

Für die Besiedlung müssen die Zellen dem Substrat zugeführt werden, ohne dass sie zerstört oder beschädigt würden, und sie müssen so auf das Substrat aufgebracht werden, daß es zu einem Anwachsen kommen kann. In den meisten Fällen wird ein geschlossener Zellrasen angestrebt werden.

Die für die Besiedlung vorgesehenen gezüchteten oder andernorts abgelösten Zellen befinden sich im allgemeinen in einem Kulturmedium. Bei herkömmlichen Besiedlungsverfahren, die hauptsächlich im Labormaßstab stattfinden, wird das Substrat mit den in dem Kulturmedium befindlichen Zellen beispielsweise in einer sogenannten Rollflasche in Kontakt gebracht. Die Rollflasche wird in einem Rollschrank gerollt, so daß das Substrat, das Kulturmedium und die Zellen in ständiger nicht zu heftiger Bewegung gehalten werden.

Bei der Besiedlung besteht das Problem, daß das Substrat einerseits von dem Kulturmedium mit den Zellen umspült werden soll, denn es sollen ständig Zellen an das Substrat herangetragen werden, damit sie dort einwachsen, andererseits jedoch darf die Strömung oder die Roll- oder Schwenkbewegung innerhalb der Flasche nicht so groß sein, daß die frisch aufgebrachten Zellen noch vor dem erfolgreichen Einwachsen wieder abgeschert werden. Darüber hinaus besteht auch das Problem, daß das Substrat für die Zellen an allen Stellen erreichbar sein soll, es darf sich also nicht durch die Rollbewegung falten oder an den Rollflachenkörper anlegen.

Bei bisherigen Besiedlungsversuchen gelang es wenig bis gar nicht, eine normale Differenzierung der anwachsenden Zellen zu erreichen. Die künstlich besiedelten Substrate unterscheiden sich daher in der Struktur ihrer Zellschichten von den natürlichen Vorbildern.

Ein weiterer Nachteil bisheriger Besiedlungsverfahren war, daß eine vollständige Konfluenz des Endothelzellrasens bei bioartifiziellen Gefäßen nicht ausgebildet werden konnte.

Es wäre daher wünschenswert, wenn ein Verfahren entwickelt werden könnte, bei dem die bei der Besiedlung verwendeten Zellen durch die Kulturbedingungen in die Lage versetzt werden gewebetypische Zellverbände und eine physiologische Differenzierung auszubilden.

Es ist außerdem aus der EP-A-320441 ein Verfahren und eine drehbare Vorrichtung zum Konditionieren von mit biologischen Zellen beschichteten Kunststoffträgern bekannt. Ausgangsmaterialien für dieses Verfahren, welches keine Besiedlungsphase umfasst, sind Substrate in Form von Elastomerkunststoffen, die bereits mit epithelialen Zellen beschichtet sind. Das Verfahren betrifft eine verbesserte Haftung von Zellen auf dem künstlichen Substrat. Die Vorrichtung ist funktionsbedingt nicht geeignet, mehrere Zelltypen separat zur Besiedlung zu bringen. Die Herausnahme des beschichteten Substrats aus der Vorrichtung ist bautechnisch bedingt mit einer erhöhten Kontaminationsgefahr verbunden.

In der WO-A-93/01843 wird ein einphasiges Verfahren und eine Vorrichtung zur Besiedlung von Substraten mit biologischen Zellen beschrieben. Die Besiedlungsvorrichtung besteht aus einem Behälter und einer darin angeordneten und mit dieser unlösbar verbundenen Einspannvorrichtung für ein zu besiedelndes Substrat. Eine sterile Herausnahme des beschichteten Substrats aus der Vorrichtung ist nicht möglich.

Der Erfindung liegt daher die Aufgabe zugrunde, ein Besiedlungsverfahren und dafür verwendbare Besiedlungsvorrichtungen zu entwickeln, mit Hilfe dessen/deren die Besiedlung eines Substrats unter optimalen Anwachsbedingungen erfolgen kann. Die zu entwickelnde Besiedlungsvorrichtung soll apparativ unaufwendig eine effektive Besiedlung ermöglichen. Ferner soll die Handhabung des Substrats während des gesamten Besiedlungsverfahrens sicher und einfach sein.

Die Besiedlungsvorrichtung soll vorzugsweise gleichzeitig als Gefäß für Sterilisation, Azellularisierung, Lagerung, Transport und Kryokonservierung verwendbar sein. Die ist erwünscht, um eine Unterbrechung der Sterilkette bis zum Endanwender zu vermeiden.

Gelöst wird diese Aufgabe durch ein Verfahren zur mehrschichtigen Besiedlung von Substraten mit biologischen Zellen entsprechend den Merkmalen des Anspruchs 1.

Unter einer Besiedlungsphase wird nach Verständnis dieser Erfindung eine solche Phase verstanden, während derer dem Substrat in geeigneter Weise Zellen zugeführt werden, mit denen das Substrat besiedelt werden soll.

Vorzugsweise werden dem Substrat während einer Besiedlungsphase Zellen in einem Medium zugeführt oder durch Aufstreichen auf das Substrat, Einbringen in das Substrat oder Beschichten des Substrates mit einer die Zellen enthaltenden Masse (z.B. Kollagen) vorgelegt.

Dabei können den Zellen Faktoren oder Co-Faktoren, insbesondere Wachstumsfaktoren oder chemotaktische Faktoren beigegeben werden.

Unter einer Perfusionsphase wird nach dem Verständnis dieser Erfindung eine solche Phase verstanden, während derer das Substrat perfundiert wird, wie dies dem üblichen fachsprachlichen Gebrauch des Begriffs "Perfusion" entspricht. Vorzugsweise wird das Substrat mit einem flüssigen Zellkultur-Nährmedium, Blut oder Plasma, die wiederum mit verschiedenen Stoffen angereichert sein können, perfundiert.

Innerhalb des Verfahrens können mehrere Besiedlungs- und Perfusionsphasen miteinander abwechseln, vorzugsweise alternierend. Während eine Durchführung der Besiedlungsphasen in ähnlicher Weise bereits bekannt war, wurde überraschenderweise gefunden, dass ein Einbeziehen ein oder mehrerer Perfusionsphasen in den Verfahrensablauf das Besiedlungsergebnis deutlich verbessert. Mit Hilfe der Perfusionsphasen werden quasi-physiologische Bedingungen simuliert, die für die Ausbildung einer normalen Zelldifferenzierung wesentlich sind. Scherstress, der durch die Perfusion in gezieltem Umfang eingeführt wird, differenziert Endothelzellen. Glatte Muskelzellen reagieren auf pulsatile Drücke, die ebenfalls während der Perfusion erzeugt werden können. Während der Perfusion können Sauerstoff und Nährstoffe herangeführt sowie Druckbelastungen ausgeübt werden. Physiologische Druckschwankungen wie sie in vivo zwischen Systole und Diastole entstehen sind von besonderer Bedeutung für die Orientierung der neugebildeten Zellen (der extrazellularen Matrix) und Ausbildung einer normalen Stabilität und Druckbelastbarkeit.
In Br. J. Surg. 1991, Vol. 78, 878-882, wurde bereits die Besiedlung eines synthetischen röhrenförmigen Gefäßes auf seiner Innenseite beschrieben. Die Besiedlung erfolgte mit Aliquots einer Endothelzellensuspension unter Drehen des Gefäßes. Dabei war festgestellt worden, daß die Besiedlung des mit Fibronectin beschichteten PTFE-Gefäßes wesentlich in ihrer Haltbarkeit gegenüber pulsierend fließendem Blut verbessert werden konnte, wenn anstelle eines kurzen (20-minütigen) Besiedlungszyklus, ein langer Zyklus über Nacht in einem Rollschrank verwendet wurde. Es wurde dort jedoch nicht erkannt, dass eine gezielte Perfusion des besiedelten Gefäßes das Besiedlungsergebnis selbst verbessern kann. Weiterhin ist das zugehörige Gerät nur um seine eigene Achse drehbar und nicht in vielen Raumrichtungen. Zusätzlich ist die Aufnahme und die Entfernung des Gefäßes sehr umständlich, da an beiden Enden Kappen entfernt werden müssen. Bei der Herausnahme des fertig besiedelten Gefäßes besteht die Gefahr, dass die Struktur unsteril wird, denn es sind einige umständliche Manipulationen erforderlich, um das Gefäß aus dem Reaktor zu entfernen.

Vorzugsweise kann die Drehung des Substrats während der Besiedlungsphasen in einer überlagerten Drehbewegung um wenigstens zwei Raumachsen gleichzeitig erfolgen. Dabei kann es sich auch um eine zufallsgesteuerte Drehbewegung in alle Raumrichtungen handeln, worauf in Zusammenhang mit den für das Verfahren geeigneten Besiedlungsvorrichtungen noch eingegangen werden wird.

Alternativ kann die Drehung des Substrates während der Besiedlungsphasen um wenigstens zwei Raumachsen nacheinander oder alternierend erfolgen.

Das Verfahren kann auch so ausgestaltet sein, dass das Substrat nach wenigstens einer Besiedlungsphase einer zusätzlichen Ruhephase unterworfen wird, während derer keine Drehung und keine Perfusion erfolgt. Eine solche Ruhephase kann zwischengeschaltet werden, um dem vollständig oder teilweise besiedelten Substrat eine belastungsfreie Konsolidierungsphase zu gönnen, während derer der Zellrasen sich festigen oder auch intern umorganisieren kann.

Die intermittierende Perfusion ermöglicht auch die Ausbildung von mehreren Schichtstrukturen; hierfür werden beispielsweise in mehreren Besiedlungsphasen hintereinander z.B. zuerst Bindegewebszellen, glatte Muskelzellen und dann Endothelzellen aufgegeben, wobei zwischen den einzelnen Besiedlungsphasen Perfusionsphasen eingeschaltet werden. Dadurch kann die Aufgabe des jeweils nachfolgenden Zelltyps auf bereits strukturierte und gewebetypisch differenzierte Verbände erfolgen.

Die Besiedlung mit Fibroblasten/glatten Muskelzellen kann über Tage und Wochen geführt werden, bis schließlich der Endothelrasen innerhalb weniger Stunden bis zu Tagen gezüchtet wird. Die Endothelzellen können zwischenzeitlich vorteilhaft kryokonserviert gelagert werden, so daß sie keine unnötige Kulturdauer erfahren.

Zusätzlich können die matrixbildenden Zellen mehrfach erneut hinzugegeben werden. Eine wiederholte Applikation von Zellen alternierend mit Perifusionsphasen ist möglich. Dies bedeutet einen Wechsel von Rotationsbewegungen der Besiedlungsvorrichtung zu Perfusionen im ruhenden Betrieb. Die Besiedlungsvorrichtung kann hierzu vertikal aber auch horizontal sowie in beliebigen anderen Positionen stationär verbleiben. Dies ist von Bedeutung bei der Erstellung von Herzklappen, wobei ein nachfolgend näher beschriebener Pulsationsbetrieb vorzugsweise in einer vertikalten Position der Herzklappe erfolgt. Hierbei ist durch die Schwerkraft ein spontaner Schluß der Klappensegel nach Beendigung der Pulsationsphase möglich.

Ferner kann das Verfahren weitere Phasen mit zusätzlichen Verfahrensschritten zur Behandlung des bereits besiedelten, teilweise besiedelten oder noch nicht besiedelten Substrats umfassen. Beispielsweise kann eine vorgeschaltete Azellularisierung des zu besiedelnden Substrates, ein oder mehrere Spülphasen, eine Sterilisierung und/oder verschiedene Begasungen in das Gesamtverfahren einbezogen sein, ohne dass der Bioreaktor geöffnet und das Substrat umgesetzt werden müßte.

In Weiterbildung der Erfindung kann zwischendurch die Applikation von Wachstumsfaktoren oder Zellen eingebettet in Hilfssubstanzen wie z.B. Hydrogelen erfolgen. Es kann z.B., insbesondere bei der Besiedlung einer Röhre, eine Kollagenmatrix (Typ I oder Mixturen mit anderen Matrixkomponenten) oder auch ein Fibrinogen oder Fibronektingemisch mit Zellen aufgegeben werden, bei einer Röhre außen und/oder innen. Dies kann durch Aufstreichen oder Auftropfen geschehen. Als zelltypspezifische Wachstumsfaktoren und chemotaktische Faktoren können beispielsweise VEGF oder PDGF appliziert werden. Hierdurch können Gradienten für das Durchwandern von Bindgewebszellen aufgebaut werden. Soweit in den verschiedenen Kammern unterschiedliche Zelltypen kultiviert werden, kann das Medium den entsprechenden Zelltypen angepasst werden.

Für die Sterilisationsphase oder Lagerphase können Unterbrechungen mit oder ohne vorausgehende oder nachfolgende Kryokonservierungsschritte erfolgen.

Die Perfusion kann vorzugsweise pulsierend erfolgen. Diese Maßnahme dient zur noch naturgetreueren Simulation physiologischer Bedingungen. Wie schon erwähnt reagieren insbesondere glatte Muskelzellen auf pulsatile Drücke, so dass sich ein derart weitergebildetes Verfahren beispielsweise für eine Besied lung mit glatten Muskelzellen anbietet. Die nachfolgend näher beschriebene Besiedlungsvorrichtung ist hierfür besonders geeignet, da das Substrat im Inneren durch Veränderung der Position der Vorrichtung in verschiedene Stellungen gebracht werden kann, außerdem wird dem Substrat genügend Raum für flexible Volumenänderungen (zu größerem oder kleinerem Volumen) gegeben.

Schließlich kann das Substrat durch das erfindungsgemäße Verfahren bereichsweise mit verschiedenen Zellen besiedelt werden, sofern dies für das jeweilige Substrat gewünscht wird. Dies ist insbesondere - wie nachfolgend noch beschrieben - mit den besonders für dieses Verfahren entwickelten Besiedlungsvorrichtungen möglich, bei denen angrenzend an das Substrat mehrere voneinander getrennte Kammern oder Kompartimente gebildet werden, die getrennt mit verschiedenen Medien oder Besiedlungs-Kulturlösungen beaufschlagt sowie auch getrennt perfundiert werden können. Ersatzweise ist es jedoch auch möglich, das Substrat bereichsweise zu schützen, z.B. abzudecken oder abzubinden, und dann verschiedene Bereiche nacheinander in verschiedenen Schritten zu behandeln.

Das Substrat kann falls gewünscht auch nacheinander mit verschiedenen Zellen besiedelt werden, wodurch auf dem Substrat mehrere Schichten mit verschiedenen Zellen gebildet werden könnten.

Das eingesetzte Substrat, das wie weiter oben bereits beschrieben vorzugsweise flächig oder röhrenförmig ist, kann selbst Innenkammern besitzen oder kann doppel- oder mehrwandig sein. Es kann auch porös oder schwammförmig sein, so dass sich Zellen in den Poren oder Kanälen dieses Material absetzen können.

Das Substrat kann aus einem biologischen Material natürlichen Ursprungs (xenogen, allogen, autolog) bestehen, aus nachgebildeten biologischen Materialien, synthetischen Proteinen, z.B. Kollagen, aus Biopolymeren, Polymeren, textilen Kunststoffen oder Feststoffen. Das Substrat kann auch ein poröser oder schwammartiger Feststoff sein, z.B. ein anorganischer Feststoff in Bimstein-Struktur. Derartige Feststoffe wären u.a. für die Herstellung von Knochenprothesen geeignet. Das Feststoffsubstrat könnte dabei stückig oder röhrenförmig ausgebildet sein. Generell können die Substrate eine beliebige Form besitzen, solange diese für ein Umströmen (ggf. innen und außen) beim Besiedeln geeignet sind.

Das Substrat kann, falls gewünscht, durch Einleiten von Kühlflüssigkeit oder Gasen wie insbesondere Stickstoff, kryokonserviert werden.

Weiterhin wird die erfindungsgemäße Aufgabe durch eine Besiedlungsvorichtung entsprechend der Merkmalen des Anspruchs 15 gelöst.

Der Behälter der Besiedlungsvorrichtung sollte so geformt sein, dass das Substrat von dem Kulturmedium frei umspült werden kann, wenn es in der Einspannvorrichtung eingespannt ist. Das Substrat kann dabei an der oder den Halterungen in jeder geeigneten Weise befestigt sein, z.B. angebunden, angeklammert oder angenäht. Die Form von Behälter und Einspannvorrichtung muß aufeinander angepasst sein. Beispielsweise kann der Behälter aus einem Gefäß mit einem Deckel bestehen, wobei der Deckel mit der Einspannvorrichtung verbunden ist und beides zusammen einen herausnehmbaren Einsatz bilden kann.

Durch einen herausnehmbaren Einsatz wird die Handhabung des Substrats vor und nach der Besiedlung vereinfacht.

Bei Herausnehmen des Einsatzes aus dem Reaktor muss das Substrat (d. h. z.B. das fertige Implantat) nicht berührt oder von feststehenden Reaktorteilen entfernt werden, die im Reaktorinneren schlecht zugänglich wären. Eine nachteilige Montage/Demontage des zu besiedelnden Substrates an fixen, nicht herausnehmbaren Gehäuseteilen wird somit vermieden. Dies erleichtert erheblich sowohl die Handhabung als auch die Verwendung nach dem Transport zum Endanwender unter sterilen Bedingungen, z.B. im Operationssaal durch den Arzt vor Implantation. Das mit der Einspannvorrichtung aus dem Reaktor herausgenommene Substrat ist frei zugänglich, bleibt vorerst stabilisiert, und kann - je nach Befestigungsmechanismus - sehr einfach abgenommen (abgebunden, abgeklemmt, abgeschnitten) werden. Die sterile Handhabung des fertig besiedelten Substrats, bzw. Implantats wird dadurch erheblich vereinfacht.

Der Einsatz ist dann in der Weise ausgebildet, dass die Einspannvorrichtung so am Behälterinneren anliegt, dass auf den entgegengesetzten Seiten des eingespannten Substrats jeweils zwei flüssigkeitstrennende Kammern gebildet werden. In den verschiedenen Kammern soll mit getrennten Medien oder Zellen gearbeitet werden können. Selbstverständlich kann auch auf beiden Seiten des Substrates, bzw. innen und außen, mit gleichen Zellen und/oder Medien gearbeitet werden. Ein wesentlicher Vorteil der Apparatur ergibt sich daraus, dass mehrfach (auch verschiedene) Zellen durchgespült werden können und dass zwischen Besiedlungs- und Perfusionsphasen gewechselt werden kann, ohne dass der Reaktor geöffnet werden müsste. Es kann auch ein gezielter Flüssigkeitstransfer durch das Substrat erwünscht sein, insbesondere bei organischen Substraten. Das zu besiedelnde Substrat ergänzt den Einsatz so, dass ein oder mehrere Kammertrennwände gebildet werden. Die Einspannvorrichtung kann auch für das Einspannen mehrerer Substrate ausgebildet sein, wobei dann mehrere Kammern gebildet werden. Jede der gebildeten Kammern ist vorzugsweise mit Flüssigkeitszu- und

-ablauf ausgestattet. Ebenso kann das Substrat selbst bereits mehrkammrig sein, insbesondere doppel- oder mehrwandig mit Lumina zwischen den Wandungen. In diesem Fall kann in Weiterbildung der Erfindung vorgesehen sein, dass die Einspannvorichtung - vorzugsweise an einer der Substrat-Halterungen - wenigstens einen weiteren Zu- und/oder Ablauf für das Einbringen von Medium, Zellen, und/oder Material in wenigstens eine Innenkammer dieses doppel- oder mehrwandigen Substrates aufweist.

Falls die Herstellung eines verzweigten Substrates gewünscht ist, kann die Einspannvorrichtung entsprechend angepasst sein, beispielsweise an einer der Halterungen verzweigt für die Herstellung einer Y-Prothese.

Alternativ kann der Behälter auch aufklappbar oder auf geeignete Weise demontierbar sein, damit das in der Einspannvorrichtung eingespannte Substrat möglichst problemlos in die Besiedlungsvorrichtung eingebracht und aus dieser wieder entfernt werden kann.

Vorzugsweise ist der Behälter der Besiedlungsvorrichtung selbst roll- oder schwenkbar ausgebildet, so daß er während der gesamten Besiedlungszeit oder für bestimmte Besiedlungsschritte in einen thermostatisierten Rollschrank eingelegt werden kann. Hierfür kann die Besiedlungsvorrichtung beispielsweise zylindrisch ausgebildet sein. Dies ist besonders vorteilhaft, wenn ein röhrenförmiges Substrat, wie eine Herzklappe oder ein anderes röhrenförmiges Gefäß besiedelt werden soll. Diese Ausführungsform wird in der Figurenbeschreibung noch näher erläutert.

Allgemein ist die Einspannvorrichtung vorzugsweise im wesentlichen quer zu einer durch eine Roll- oder Schwenkbewegung des Behälters vorgegebenen Vorzugsbewegungsrichtung eines darin befindlichen flüssigen Mediums angeordnet. Die Einspannvorrichtung kann dabei vorzugsweise in Bezug auf die Zu- und Abläufe so angeordnet sein, daß eine durch die Zu- und Abläufe vorgegebenen Strömungsrichtung des flüssigen Mediums durch den Reaktor längs oder innerhalb des eingespannten Substrats verläuft.

Für flache Substrate, beispielsweise Hautstücke, kann die Einspannvorrichtung aus wenigstens einer rahmenförmigen Halterung bestehen. Die rahmenförmige Halterung wäre dann vorzugsweise seitlich an einem Einschubteil befestigt, das gleichzeitig einen Deckel für einen kastenförmigen Behälter bildet. Bei Einsetzen des Einsatzes mit der rahmenförmigen Halterung sollte der Rahmen an drei Seiten innen an dem kastenförmigen Behälter anliegen, während die vierte Seite mit dem am Einsatz befestigten Deckel verbunden ist. Hierdurch ergeben sich innerhalb des Behälters drei Kammern, die jeweils mit einer Zu- und Ableitung für flüssiges Medium versehen sein müssen. Diese Zu- und Ableitungen können über den Deckel des Einsatzes geführt werden. Da ein kastenförmiger Behälter selbst nicht rollbar ist, würde dieser für den Einsatz im Rollschrank in eine Halterung eingesetzt. Eine derartige Anordnung ist weiter unten beschrieben.

Die Einspannvorrichtung kann alternativ auch aus zwei ringförmigen Halterungen für das Substrat bestehen, die mechanisch fest untereinander verbunden sind, beispielsweise durch Stangen.

Der roll- oder schwenkbare Behälter kann auch kugelförmig oder kugelähnlich geformt sein, um weitere Freiheitsgrade bei der Bewegung im Rollschrank zu ermöglichen. Dabei können zusätzlich noch Auslenknoppen an dem kugelförmigen Behälter (bzw. der kugelförmigen Halterung, siehe unten) vorgesehen sein, wodurch die Rollrichtung bei Behandlung in einem Rollschrank oder einem entsprechenden Apparat noch häufiger zufallsgesteuert geändert wird und ein Drehen und Schwenken in allen Raumrichtungen möglich ist. Der Behälter kann aus einem starren oder einem flexiblen Material sein. Letzteres bringt u.a. den Vorteil mit sich, dass es ein in gewissen Umfang variables Volumen zulässt, so dass sich das Substrat z. B. bei Druckbelastung noch stärker ausdehnen kann.

Zusätzlich zu den Flüssigkeitszu- und abläufen, durch die bei Besiedlung das Medium mit den Zellen geführt wird, können die Kammern mit ein oder mehreren Gaszu- und Gasabführleitungen ausgestattet sein. Die Gaszu- und Abführleitungen können die Form von Schläuchen haben, die an den Wänden entlang geführt werden und aus gaspermeablen Materialien bestehen, wie z.B. Silikon. Durch diese können dem Kulturmedium während der Besiedlung Gase zugeführt werden bzw. von diesem abgezogen werden. Der Gasaustausch kann jedoch - ersatzweise oder zusätzlich - auch durch gasdurchlässige Außenwände des Behälters der Besiedlungsvorrichtung ermöglicht werden.

Hierzu können die Wände des Behälters aus einer PTFE-Folie oder einer Silikonfolie gebildet sein oder Einsätze aus diesen Strukturen oder gaspermeablen Materialien enthalten. Die Wandstrukturen können auch aus Sintermetall bzw. einer mikroporösen Keramik oder porösem Glas gebildet sein. Diese poröse Trägerstruktur kann mit einer gaspermeablen Schicht, z.B. einer Folie aus Kunststoff (z.B. PTFE, Silikon) überzogen sein.

Ein Vorteil der in die Wandstrukturen des Gehäuses der Besiedlungsvorrichtung integrierten gaspermeablen Membranen oder Folien ist, dass die Umgebungsatmosphäre im Brutschrank(herkömmlicher Rollschrank oder gesondert dafür vorgesehene Apparatur) direkt für den Sauerstoffaustausch und über eine Kohlendioxid-Phase auch für die pH-Regulation in dem Kulturmedium eingesetzt werden kann. Von Bedeutung hierbei ist, dass somit den Besiedlungskompartimenten (-kammern) einer eingesetzten dreidimensionalen Struktur (z.B. Gefäß oder Herzklappe) gleichzeitig ein Kulturreservoir und eine Oxygenierungsfunktion zur Verfügung steht.

Die Zu- und Abläufe des Nährmediums bzw. die Gaszu- und - Abführleitungen können an dem Besiedlungsvorrichtung mit drehbaren Kupplungen angeschlossen sein, wodurch es ermöglicht würde, dass die Besiedlung während des Rollens des Besiedlungsvorrichtung in einem besonders hierfür ausgelegten Rollschrank durchgeführt werden kann. Hierfür könnten die über die Kupplung angeschlossenen Leitungen konzentrisch ausgeführt sein.

Schließlich ist es in Weiterbildung der Erfindung möglich, dass im Inneren des Behälters bestimmte Einbauten angeordnet sind, die das flüssige Medium, während es durch den Reaktor strömt, in gewünschter Weise leiten. Hierbei kann es sich um an der Behälterinnenwand angeordnete Stege oder Schläuche handeln. Geeignete Einbauten im weiteren Sinne sind aus dem Reaktorbau für andere Zwecke bekannt.

Der Reaktorbehälter kann auch mit als solches bekannten Vorrichtungen zum Heizen oder Kühlen versehen oder verbunden sein. So können z. B. in die Außenwand des Reaktors Heizdrähte zum Temperieren des Mediums eingelassen sein. Auch kann der Reaktorbehälter für die Aufnahme eines Heiz- oder Kühlmediums, wie z. B. Wasser, ummantelt sein.

Über die Zu- und Abläufe wird das Kulturmedium durch den Reaktor geleitet, das die zu besiedelnden Zellen enthält. Alternativ kann auch eine bestimmte Menge an Zellen in dem Reaktor vorgegeben werden, wobei dann nur das reine Kulturmedium umläuft. Die Zu- und Abläufe werden vorzugsweise mit Filtern versehen sein, um ein ungewolltes Ausschwämmen der Zellen zu verhindern. Zusätzlich sind an den Zu- und Abläufen Verschlüsse (Absperrventile) vorgesehen, um die einzelnen Kammern verschließen zu können. Die Rezirkulation von Zellen wird bei dem erfindungsgemäßen Verfahren vorzugsweise vermieden. Im Gegensatz zu der früheren Flußbesiedlung werden hier die Zellen über dem Substrat gehalten.

Wie bereits oben geschildert, befindet sich im Inneren des Behälters eine Einspannvorrichtung für das zu besiedelnde Substrat. Das Substrat wird während der Besiedlung in einer Einspannvorrichtung gehaltert, damit es von den gewünschten Seiten für das Kulturmedium und die Zellen frei zugänglich ist und nicht in sich zusammenfällt oder aneinander verklebt.Gleichzeitig dient die Halterung dazu, das Substrat innerhalb des vorzugsweise herausnehmbaren Einsatzes in den Reaktor einzuführen und aus diesem wieder zu entnehmen. Der herausnehmbare Einsatz dient damit gleichzeitig einer verbesserten Handhabung des Substrats, das darin beispielsweise unmittelbar nach der Besiedlung in eine Flüssigkeit eingelegt, in sonstiger Weise behandelt oder konserviert werden kann.

Da das zu besiedelnde Substrat die Fläche zwischen Zu- und Ablauf innerhalb der Einspannvorrichtung kontinuierlich verbinden muß, können zusätzlich zur Längen- und Größenadaption Zwischenstücke aus z.B. mit Fasern armierten Röhren oder Trichtern aus Silikon oder anderen vorzugsweise elastischen Kunststoffen eingebracht werden. Diese können verschieblich hinsichtlich Distanzüberbrückungen als auch Verkürzungen sein.

Die Einspannvorrichtung ist erfindungsgemäß im wesentlichen quer zu einer durch die Roll- oder Schwenkbewegung des Behälters bzw. des in der unten noch beschriebenen Halterung gehalterten Behälters vorgegebene Vorzugsbewegungsrichtung des flüssigen Mediums angeordnet. "Im wesentlichen quer" soll bedeuten, dass das Substrat mit Hilfe der Einspannvorrichtung so angeordnet ist, dass das bewegte flüssige Medium, d.h. die Zellsuspension in Kulturmedium, durch das Rollen oder Schwenken stetig in einem Winkel zwischen etwa 30 und 150° zur Drehachse der Roll - oder Schwenkbewegung auf das Substrat "aufgeworfen", d.h. mit diesem immer wieder in Kontakt gebracht wird. Dabei wird die Vorzugsbewegungsrichtung des flüssigen Mediums unter Vernachlässigung einer womöglichen(aber nicht zwingend) gleichzeitigen Strömung des flüssigen Mediums längs des eingespannten Substrats betrachtet.

Bei der Besiedlung, d.h. zumindest während bestimmter Besiedlungsperioden, wird der roll- oder schwenkbare Behälter, d.h. die Besiedlungsvorrichtung im Ganzen, in einen Rollschrank oder eine Apparatur zum Schwenken oder in vergleichbare Vorrichtungen mit entsprechender Funktion eingebracht und dort gerollt oder geschwenkt, um das Kulturmedium und die darin suspendierten Zellen relativ zu dem eingespannten Substrat zu bewegen.

Die Besiedlungsvorrichtung wird in dem Rollschrank ebenso wie eine Rollflasche behandelt, d.h. gedreht und ggf. temperiert. Anstelle des Rollschrankes kann jedoch auch eine andere Vorrichtung verwendet werden, die geeignet ist, die Besiedlungsvorrichtung in der gewünschten Weise zu bewegen. Dies kann auch eine speziell für diesen Zweck vorgesehene gesonderte Apparatur sein. Der Rollschrank bzw. die Apparatur können auch eine spezifische Atmosphäre (Sauerstoff, Kohlendioxid) zur Verfügung stellen.

Das Durchströmen des Reaktors mit Kulturmedium bzw. Zellsuspension durch den Flüssigkeitszu- und -ablauf oder auch durch mehrere Zu- und Abläufe muß nicht während des gesamten Besiedlungsvorgangs erfolgen. Es kann auch besonders zweckmäßig sein, diesen Strom zeitweilig - beispielsweise während des Rollens im Rollschrank oder der Behandlung in einer Vorrichtung zu dem entsprechenden Zweck - anzuhalten. Hierzu können die Zu- und Abläufe zeitweilig abgeklemmt oder mit Hähnen verschlossen werden.

Das Kulturmedium kann - mit oder ohne Zellen - wenigstens zeitweilig durch die Zu- und Abläufe im Kreis geführt werden. Hierdurch werden Zellen, die im ersten Durchgang nicht anwachsen konnten, dem Substrat nochmals angeboten.

Die in dem Kulturmedium beweglichen noch nicht angewachsenen Zellen werden mit dem Längsstrom durch den Zulauf dem Substrat einerseits zugeführt und zweitens längs des Substrates verteilt. Die Verteilung "quer" zu dieser Längsrichtung erfolgt im wesentlichen durch die Roll- oder Schwenkbewegung, wobei beide Bewegungen sich überlagern können und dann zusammenwirken. Die Roll- oder Schwenkbewegung kann für den Zweck, die Zellen immer wieder mit dem Substrat in Kontakt zu bringen und auf diesem zu verteilen, noch mit anderen Arten der Bewegung überlagert sein, z.B. mit einer Rüttelbewegung.

Dadurch, dass eine Strömung entlang des Substrats eingestellt werden kann, wird eine gleichmäßige Zufuhr von Zellen zu allen Stellen des Substrats ermöglicht. Die Strömungsgeschwindigkeit kann so eingestellt werden, dass der Transport der Zellen, deren Einwachsen nach Möglichkeit nicht behindert. Das Einwachsen kann auch dadurch begünstigt werden, dass der Kulturmediumstrom eine Zeit lang angehalten wird, so dass die gerade über dem Substrat befindlichen Zellen dort eine Zeit verbleiben und Gelegenheit haben an- bzw. einzuwachsen.

Die Erfindung umfaßt weiterhin eine Anordnung aus einer Besiedlungsvorrichtung wie vorstehend beschrieben und einer rollbar ausgebildeten Halterung. Die Halterung wird verwendet, um einen als solchen nicht rollbar Behälter, wie z.B. einen kastenförmigen Behälter in einem Rollschrank behandeln zu können, oder um zusätzliche Bewegungsrichtungen einzuführen. Vorzugsweise verlaufen die Rotationsachse der rollbaren Halterung und eine Längsachse des in der Einspannvorrichtung des Besiedlungsvorrichtungs eingespannten Substrats in einem Winkel zueinander.

Als Halterung für die vorgenannte Anordnung kann ein rotationssymmetrischer Körper verwendet werden, der eine oder mehrere Ausnehmung(en) oder Aufnahme(n) für eine Besiedlungsvorrichtung, wie vorstehend beschrieben, aufweist. Dieser rotationssymmetrische Körper kann aus einem geeigneten Material, wie z.B. Glas, Kunststoff oder Metall bestehen. Beispielsweise kann die Halterung aus einer Kugel mit einem röhrenförmigen Durchlaß bestehen. In diesen röhrenförmigen Durchlaß kann dann ein zylinderförmiger Reaktionsbehälter eingesetzt werden (es können auch mehrere Röhren für mehrere Behälter vorhanden sein), und die Kugel kann, nach Abklemmen der Zu- und Abführungen von der Besiedlungsvorrichtung, in einen Rollschrank eingelegt werden, so dass das Substrat während des Rollens in alle Richtungen gewendet wird. Die Halterung kann auch aus einem zylinderförmigen Körper oder Gestell bestehen, in dem ein röhrenförmiger Durchlaß schräg zur Zylinderachse angeordnet ist. Bei Rollen der Besiedlungsvorrichtung in dieser Halterung wird der Behälter und damit das Substrat einer zusätzlichen Schwenkbewegung quer zu seiner Längsachse unterworfen.

Nach Besiedlung des Substrates ist die Besiedlungsvorrichtung auch geeignet, um als Transport- und Lagerbehälter für das fertig besiedelte Substrat zu dienen. Hierdurch wird vermieden, dass das empfindliche besiedelte Substrat weiter unmittelbar gehandhabt, z.B. aus der Kulturlösung entnommen werden müßte. Es kann vielmehr bis zum Gebrauch in der Besiedlungsvorrichtung verbleiben. Die Zu- und Abläufe sowie Gaszu- und - abführleitungen können für Transportzwecke abgeklemmt werden. Ansonsten ist es möglich, das besiedelte Substrat in dem gewünschten (ständig erneuerbaren) Medium unter einer beliebig wählbaren Atmosphäre zu belassen.

Das Substrat kann auch in der Besiedlungsvorrichtung gekühlt werden. Durch Einleiten von Kühlflüssigkeiten bzw. -gasen (wie z.B. Stickstoff) in zumindest eine oder besser gleichzeitig alle Kammern kann eine Kryokonservierung eines besiedelten oder noch nicht besiedelten Substrates erreicht werden. Für Lagerungszwecke kann eine Kryokonservierung auch vor Besiedlung des Substrates erfolgen. Dies kann z.B. an eine Sterilisationsphase angeschlossen werden.

An der Innenwand des Behälters der Besiedlungsvorrichtung kann in Weiterbildung der Erfindung mit Hilfe einer mikroporösen Trennwand eine zusätzliche Kammer mit wenigstens einem Zu- und Ablauf gebildet werden. Auf gleiche Weise können auch mehrere Kammern gebildet werden. In diesen Kammern können besondere Medien oder Kulturen (sog. feeder-Kulturen) vorgehalten werden, die eine oder mehrere Besiedlungskammern der Vorrichtung mit bestimmten Nährstoffen oder Faktoren versorgen.

Im folgenden wird die Erfindung anhand von Ausführungsbeispielen näher erläutert.
- Fig. 1: zeigt eine zylindrische Zweikammer-Besiedlungsvorrichtung für ein röhrenförmiges Substrat in einem Längsschnitt in Explosionsdarstellung;
- Fig. 2: zeigt eine Vorrichtung, wie in Figur 1 dargestellt, mit einer zusätzlichen durch eine poröse Trennwand an der Behälterinnenseite ausgebildeten Kammer;
- Fig. 3: zeigt eine zylindrische Zweikammer-Besiedlungsvorrichtung für ein doppelwandiges röhrenförmiges Substrat in einer Fig.1 entsprechender Darstellung;
- Fig. 4: zeigt ein mehrwandiges Substrat im Längsschnitt(ohne den Reaktor)
- Fig. 5: zeigt eine Teilansicht einer Einspannvorrichtung für ein verzweigtes Substrat (ebenfalls in Teilansicht dargestellt;
- Fig. 6: zeigt einen Querschnitt durch eine kastenförmigen Besiedlungsvorrichtung mit einer rahmenförmigen Einspannvorrichtung;
- Fig. 7: zeigt eine Halterung für eine zylindrische Besiedlungsvorrichtung nach der Erfindung

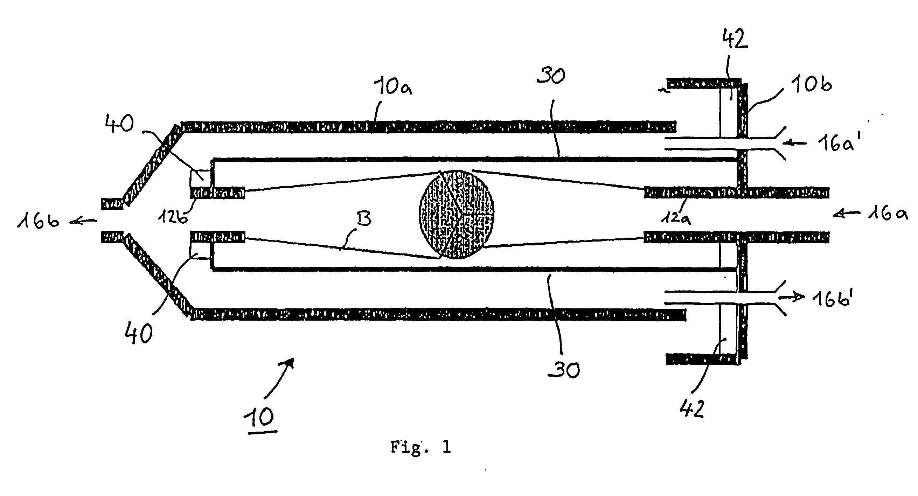

Fig. 1 zeigt ein erstes Ausführungsbeispiel der Erfindung im Längsschnitt in einer Explosionsdarstellung. Es handelt sich um eine zylinderförmige Besiedlungsvorrichtung, die für die Besiedlung eines röhrenförmigen Substrates, in der Zeichnung mit B bezeichnet, ausgebildet ist. Der Behälter 10 besteht aus zwei Behälterteilen 10a, 10b, die in der Darstellung nicht zusammengesteckt sind. Der Flüssigkeitszulauf 16a bildet im Inneren der Vorrichtung gleichzeitig eine erste ring- bzw. röhrenförmige Halterung 12a für das Substrat. Die zweite ringförmige Halterung 12b ist über Stangen 30 mit dem Behälterdeckel 10b und damit mechanisch fest mit der ersten Halterung 12a verbunden. Die Halterungen 12a, 12b bilden zusammen die Einspannvorrichtung für das röhrenförmige Substrat B, eine Herzklappe. Beim Zusammenschieben der Behälterteile 10a und 10b dichtet die Halterung 12b über den Dichtungsring 40 gegen die Innenwand des Behälterteils 10a und den Abfluß 16b ab. Gleichzeitig wird der Behälter 10 aus den Behälterteilen 10a und 10b geschlossen und mit der Dichtung 42 abgedichtet. Hierdurch entstehen, unter Mitwirkung eingespannten Substrates B, zwei Kammern, eine im Innern des röhrenförmigen Substrates B und eine um das Substrat herum. Für ein Zuführen von Zellen zum Substratinneren wird die ZellSuspension und/oder das Kulturmedium durch den Zulauf 16a zugeführt, läuft innen durch die ringförmigen Halterungen 12a und 12b sowie das Substrat und wird durch den Ablauf 16b wieder abgeführt. Während der Besiedlungsphase werden der Zulauf 16a und der Ablauf 16b geschlossen gehalten. Nach Beendigung der Besiedlungsphase wird das Medium über den Ablauf 16b wieder abgeführt. Die Zellsuspension und/oder das Kulturmedium für die Besiedlung der äußeren Oberfläche des Substrats wird durch den Zulauf 16a zugeführt, verteilt sich im Behälterinneren um das Substrat B herum und wird über den Ablauf 16b wieder abgeführt. Ansonsten erfolgt die Besiedlung wie bereits beschrieben.

Während der Perfusionsphase können die beiden getrennten Kammern bzw. Kompartimente getrennt perfundiert werden. Die Perfusion kann mit unterschiedlichen Wachstumsfaktoren und Konzentration erfolgen, so dass unterschiedliche Medien zelltypspezifisch angeboten werden und interne Gradienten ausgebildet werden können. Besiedlungs- und Perfusionsphase können sich abwechseln, ohne dass der Reaktor geöffnet werden müsste.

Am Ende der Behandlung kann der Einsatz mit dem Substrat beim Abnehmen des Deckels insgesamt herausgezogen werden wie eine Schublade. Die Handhabung des fertigen Substrats, bzw. hier Herzklappen-Implantats wird dadurch erheblich vereinfacht, und die Sicherheit bezüglich Sterilität und Unversehrtheit des Substrats erhöht.

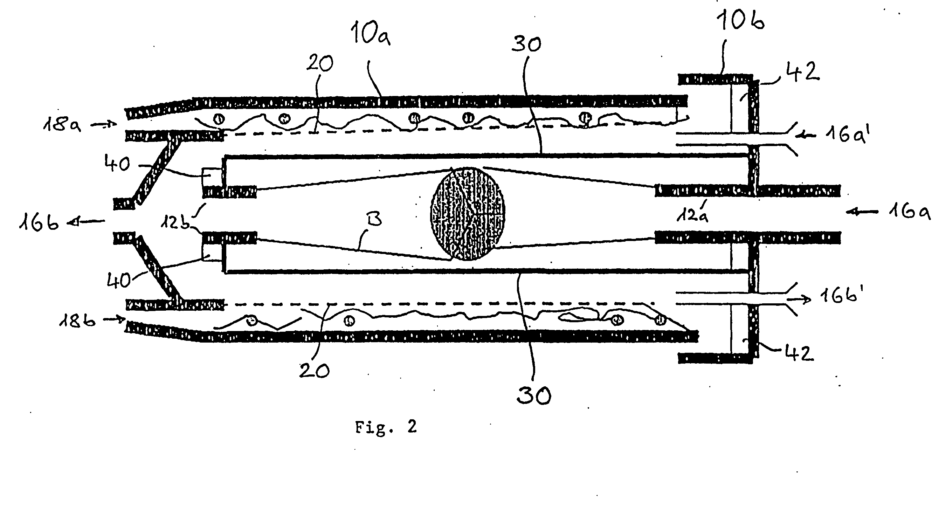

Figur 2 zeigt eine Vorrichtung wie in Figur 1 gezeigt, bei der an der Innenwand des zylindrischen Behälters 10a, d.h. innerhalb des Zylindermantels, durch eine zusätzliche mikroporöse Trennwand 20 eine zusätzliche Kammer abgetrennt ist. Diese zusätzliche Kammer kann durch wenigstens einen Zulauf 18a und wenigstens einen Ablauf 18b mit dem gewünschten Medium beschickt werden. Mittels der mikroporösen Trennwand besteht ein Austausch zwischen der zusätzlichen Kammer und dem Behälterinneren. Innerhalb der zusätzlichen Kammer könnten daher Nährstoffe oder bestirnte Gase erzeugt werden, die dann durch Kommunikation mit dem Behälterinneren einem Substrat zugeführt werden können. Die mikroporöse Trennwand kann z.B. aus einer Silikonfolie, aus PTFE, Sinterglas, Polycarbonat, Cellulose, Polyamiden oder anderen Kunststoffen bestehen.

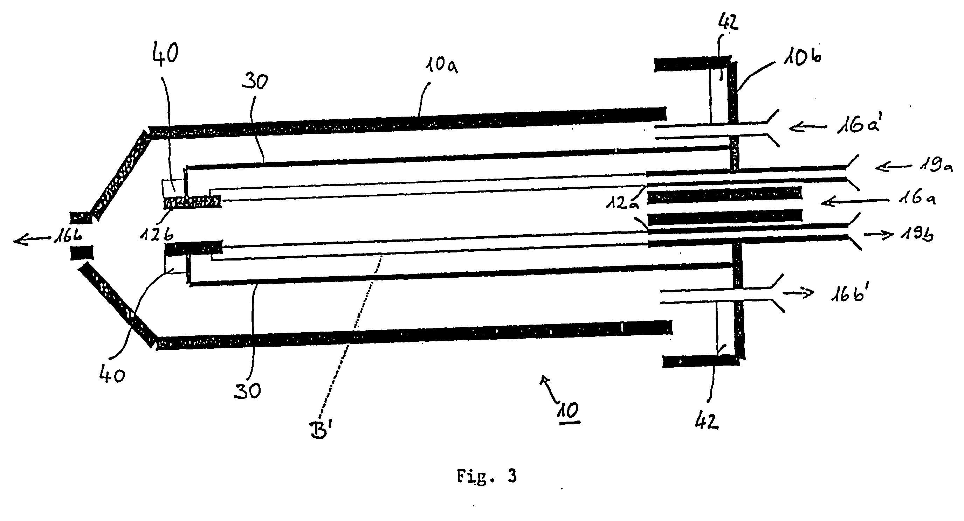

Figur 3 zeigt eine zylindrische Zweikammer-Besiedlingsvorrichtung 10 für ein doppelwandiges röhrenförmiges Substrat B'. Der Besiedlungsreaktor gleicht weitgehend dem im Figur 1 gezeigten Beispiel, wobei gleiche Bezugszeichen gleiche Bauteile bezeichnen. Der Unterschied zu der Besiedlungsvorrichtung aus Figur 1 besteht darin, dass dieser Reaktor für ein wenigstens doppelwandiges Substrat geeignet ist und zwei zusätzliche Zu- und Abläufe 19a, 19b aufweist, mit deren Hilfe ein Medium oder Material in ein Lumen zwischen die Wände des doppelwandigen Substrates B' eingeführt werden kann. Diese Zuläufe/Abläufe 19a/19b sind an der einen Halterung 12a der Einspannvorrichtung angeordnet. Die Halterungen 12a und 12b haltern das röhrenförmige, jedoch doppelwandige Substrat an beiden Enden, wobei das Substratende an der Halterung 12b zusammengefasst bzw. zusammengebunden ist, während an der Halterung 12a die Zu- und Abführung 19a, 19b zwischen die beiden Wände des Substrates eingeführt wird. Das Innere des Substrats kann dann mit dem gewünschten Medium oder Material sowie auch mit weiteren Zellen versorgt werden.

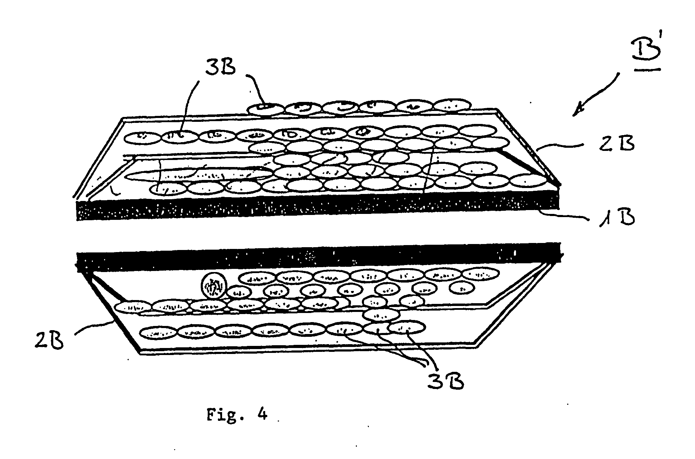

Figur 4 zeigt ein weiteres Ausführungsbeispiel eines solchen mehrwandigen Substrates B' - im vorliegenden Fall handelt es sich um ein dreiwandiges Substrat. Das Substrat kann hier vorzugsweise auch ein synthetisches Substrat sein. Im vorliegenden Beispiel wurde die innere Wandung des vorliegenden Substrates mit 1B bezeichnet. Darüber sind zwei weitere Hüllen 2b angeordnet, die beliebige Materialien oder Zellen in Schichten auf dem inneren Substrat halten. Das Substrat ist hier an seinen Enden zusammengeführt, so dass es gemeinsam auf den nicht dargestellten Halterungen 12a, 12b befestigt wird.

Die Kommunikation mit dem Nähr- oder Besiedlungsmedium erfolgt daher nicht wie in Figur 3 über die Zu- und Abläufe 19a, 19b, sondern mittels Diffusion quer zur Strömungsrichtung. In diesem Beispiel sind Zellen 3B gezeigt, die in und auf das Substrat ein- bzw. aufgebracht sind. Die mehrschichtige Substratstruktur ermöglicht deutlich dickere Substrate, so dass sich beispielsweise Gewebe unter dem lokalen Differenzierungsdruck naturähnlich kapillarisiert ausbilden können.

In einem weiteren Ausführungsbeispiel könnte das Substrat aus einem festen, insbesondere einem festen anorganischen Material bestehen. Zum Beispiel kann als anorganisches Feststoffmaterial ein calciumhaltiges Feststoffmaterial, insbesondere tricalciumphosphathaltiges Feststoffmaterial, z. B. als ggf. gesintertes oder geklebtes Pressgranulat verwendet werden. Gerade in ein solches poröses Feststoffmaterial kann mit dem erfindungsgemäßen Bioreaktor sehr gut Material in die poröse Wandstruktur des Feststoffs eingebracht werden.

Ein großer Vorteil der Bioreaktortechnik besteht in jedem Falle darin, dass die als Implantate vorgesehenen Produkte erst im Operationssaal unmittelbar vor der Implantation aus dem Bioreaktor entnommen werden, so dass von der ersten Behandlung des Substrats bis zur Implantation des besiedelten Substrats in einem Patienten eine geschlossene sterile Handhabung gewährleistet ist.

Besonders vorteilhaft können in dem erfindungsgemäßen Bioreaktor röhrenförmige Knochenimplantate hergestellt werden, da eine Besiedlung des Röhreninneren getrennt vom Röhrenäußeren möglich ist.

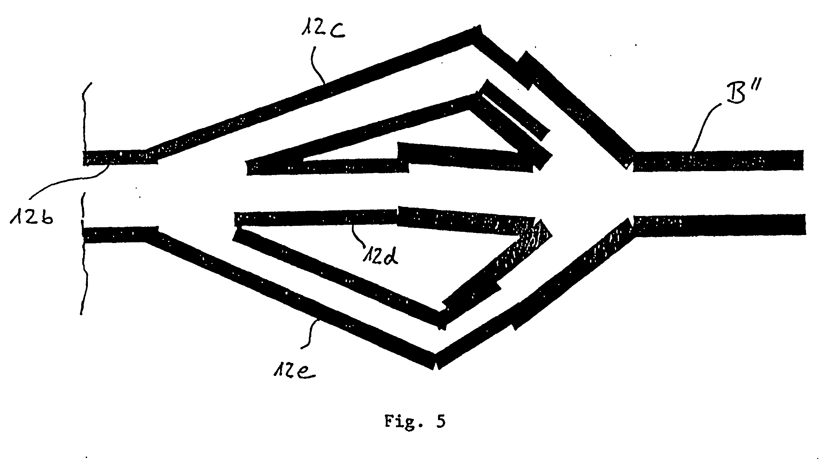

Figur 5 zeigt eine Teilansicht einer Einspannvorrichtung für ein verzweigtes Substrat, das selbst ebenfalls in einer Teilansicht dargestellt ist. Die Halterung 12b der Einspannvorrichtung verzweigt sich im vorliegenden Fall in die röhrenförmigen Halterungsabschnitte 12c, 12d und 12e, die jeweils einen Zweig einer Y-Prothese B" - im vorliegenden Falle mit einem in drei Äste verzweigten Ende - haltern. Im übrigen kann die Vorrichtung, wie in den Figuren 1 bis 3 dargestellt, ausgebildet sein.

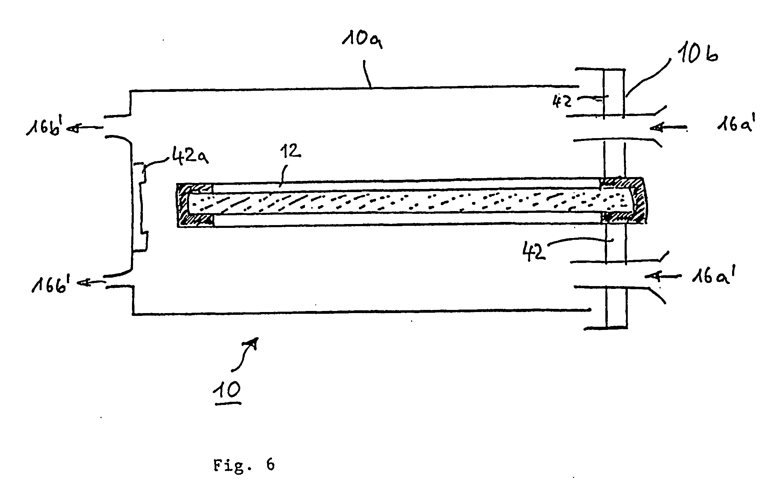

Figur 6 zeigt ein weiteres Ausführungsbeispiel eine Besiedlungsvorrichtung aus einem kastenförmigen Behälter 10a mit rechteckigem Deckel 10b. In diesem Behälter 10 ist eine rahmen-förmige Einspannvorrichtung 12 angeordnet, die mit dem Deckel 10b fest verbunden ist. Das Abdichten des Einsatzes aus Einspannvorrichtung und Deckel gegen den Behälter 10a erfolgt mit Hilfe der Dichtungen 42, wobei die Dichtung 42a eine im Behälterinneren um die rahmenförmige Einspannvorrichtung 12 umlaufende U-förmige Dichtung ist, die in geeigneter Weise profi-liert sein kann. Im zusammengebauten Zustand werden innerhalb des Besiedlungsvorrichtungs zwei flüssigkeitstrennende geschlossene Kammern gebildet, eine oberhalb und eine unterhalb des Substrates.

In Abhängigkeit der Porosität des zu besiedelnden Substrates kann ein interner Flüssigkeitstransfer zwischen den Kammern erfolgen und gewünscht sein. Hierdurch kann erreicht werden, daß oxygeniertes Medium während der Rollphase der Besiedlungsvor- richtung auch in die Innenstrukturen gelangen kann.

Jede der Kammern ist mit einem Flüssigkeitszulauf 16a, 16a' und mit einem Flüssigkeitsablauf 16b, 16b' ausgestattet. Zusätzlich können Gaszu- und Abführungsleitungen vorgesehen sein, die hier nicht dargestellt sind. Solange der Reaktor während der Besiedlung gerollt wird, werden die Zu- und Abführleitungen 16a, 16a, 16b, 16b' abgeklemmt. Ist dies nicht gewünscht so sind drehbare Kupplungen vorgesehen. Üblicherweise wird jedoch stufenweise vorgegangen.

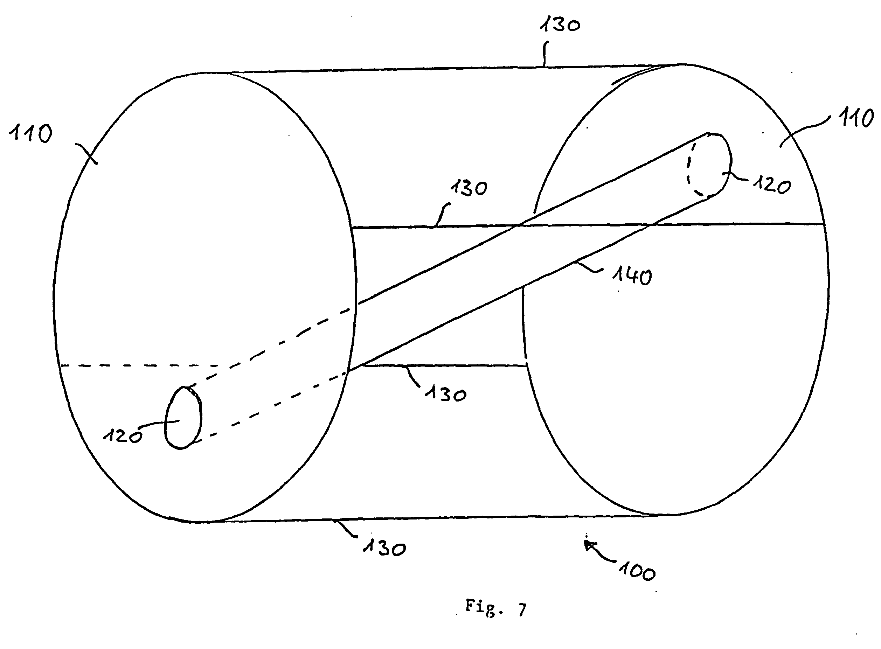

Figur 7 zeigt eine geeignete rollbare Halterung 100, in der die Besiedlungsvorrichtung 10 während der Besiedlung in einem Rollschrank zusätzlich geschwenkt werden kann. Die Halterung 100 besteht aus zwei Scheiben 110 aus Glas oder Plexiglas, die azentrisch mit zwei Öffnungen 120 versehen sind. Für den Einsatz einer zylindrischen Besiedlungsvorrichtung 10 sind die Öffnungen 120 am besten oval oder zylinderschnittförmig; die Scheiben 110 sind in diesem Beispiel kreisförmig, können jedoch auch anders, z.B. eliptisch, geformt sein. Die Scheiben 110 sind im vorliegenden Beispiel durch mehrere Stangen 130 verbunden, sie könnten jedoch auch durch einen geschlossenen Zylindermantel verbunden sein. Die Verbindung der Scheiben 110 ist so gewählt, daß sich die Öffnungen 120 in Projektion versetzt gegenüberliegen. Zwischen den Öffnungen 120 ist eine Röhre 140 eingesetzt, in die die Besiedlungsvorrichtung 10 eingeschoben werden kann. Die Längsachse der in der Zeichnung nicht dargestellten Besiedlungsvorrichtung verläuft dann schräg zur Längsachse der insgesamt zylindrischen Halterung 100. Die Halterung 100 bildet mit der Besiedlungsvorrichtung 10 zusammen eine Anordnung, die in einen Rollschrank eingelegt werden kann. Bei Behandlung im Rollschrank wird die Besiedlungsvorrichtung einer Rollbewegung um ihre Längsachse und gleichzeitig einer Schwenkbewegung quer zu ihrer Längsachse ausgesetzt. Hierdurch kann das Kulturmedium besonders intensiv und gleichmäßig verteilt mit dem Substrat in Kontakt gebracht werden.

Die Seitenwände der in den Figuren gezeigten Vorrichtungen (zylindermantelförmige, rechteckige, kugelförmige Außenwände) können z.B. aus gasdurchlässigen PTFE- oder Silikonfolien bestehen, um eine Verbesserung der Sauerstoffversorgung während perfusionsfreier Besiedlungsphasen zu erreichen. Ein ständiger Gasaustausch oder eine zeitweilige bewußte Oxygenierung mit Sauerstoff oder Sauerstoffgemisch erhöht erheblich die Adhäsionseffizienz (bis 100-200 %) während der Rollphase. Somit wird das Substrat zu allen Zeiten (Rollphase und Perfusionsphase) optimal mit Sauerstoff versorgt. Durch die Umwälzung gelangt verbrauchtes Medium kontinuierlich an die oxygenierenden Reaktorwände.

## Patentansprüche

1. Verfahren zur mehrschichtigen Besiedlung von Substraten (B) mit biologischen Zellen (3B), wobei innerhalb einer Vorrichtung (10) ein darin gehaltertes Substrat sowie für die Besiedlung vorgesehene Zellen (3B) wenigstens einer Besiedlungsphase unterworfen werden, während welcher das Substrat (B) in oder mit der Vorrichtung (10) um wenigstens eine Achse gedreht wird und die Zellen (38) während der Drehbewegung immer wieder in Kontakt mit dem Substrat (B) gebracht werden, **dadurch gekennzeichnet, dass**
- das Substrat (B) nach oder während einer oder mehreren Besiedlungsphasen wenigstens einer Perfusionsphase unterworfen wird, während welcher das Substrat (B) wenigstens an Bereichen seiner äußeren oder inneren Oberfläche perfundiert wird.

2. Verfahren nach Anspruch 1, **dadurch gekennzeichnet, dass** während einer Besiedlungsphase Zellen (3B) in einem Medium zugeführt oder durch Aufstreichen auf das Substrat (B), Einbringen in das Substrat (B) oder Beschichten des Substrates (B) mit einer die Zellen (3B) enthaltenden Masse vorgelegt werden.

3. Verfahren nach Anspruch 2, **dadurch gekennzeichnet, dass** den Zellen (3B) Faktoren oder Co-Faktoren, insbesondere Wachstumsfaktoren oder chemotaktische Faktoren beigegeben werden.

4. Verfahren nach einem der Ansprüche 1 bis 3, **dadurch gekennzeichnet, dass** während einer Perfusionsphase das Substrat (B) mit einem flüssigen Medium, vorzugsweise einem Zellkultur-Nährmedium oder Blut, perfundiert wird.

5. Verfahren nach einem der Ansprüche 1 bis 4, **dadurch gekennzeichnet, dass** die Drehung in einer überlagerten Drehbewegung um wenigstens zwei Raumachsen gleichzeitig erfolgt.

6. Verfahren nach einem der Ansprüche 1 bis 4, **dadurch gekennzeichnet, dass** die Drehung um wenigstens zwei Raumachsen nacheinander oder alternierend erfolgt.

7. Verfahren nach einem der Ansprüche 1 bis 6, **dadurch gekennzeichnet, dass** die Besiedlungs- und Perfusionsphasen alternierend erfolgen.

8. Verfahren nach einem der Ansprüche 1 bis 7, **dadurch gekennzeichnet, dass** das Substrat (B) nach wenigstens einer Besiedlungsphase einer zusätzlichen Ruhephase unterworfen wird, während derer keine Drehung und keine Perfusion erfolgt.

9. Verfahren nach einem der Ansprüche 1 bis 8, **dadurch gekennzeichnet, dass** das Verfahren weitere Phasen mit zusätzlichen Verfahrensschritten zur Behandlung des bereits besiedelten, teilweise besiedelten oder noch nicht besiedelten Substrats (B) umfasst.

10. Verfahren nach einem der Ansprüche 1 bis 9, **dadurch gekennzeichnet, dass** die Perfusion pulsierend erfolgt.

11. Verfahren nach einem der Ansprüche 1 bis 10, **dadurch gekennzeichnet, dass** das Substrat (B) bereichsweise mit verschiedenen Zellen (3B) besiedelt wird.

12. Verfahren nach einem der Ansprüche 1 bis 10, **dadurch gekennzeichnet, dass** das Substrat (B) nacheinander oder an verschiedenen Positionen mit unterschiedlichen Zellen (3B) besiedelt wird.

13. Verfahren nach einem der Ansprüche 1 bis 12, **dadurch gekennzeichnet, dass** das Substrat (B) in der zur Behandlung dienenden Vorrichtung (10) zusätzlich azellularisiert und/oder sterilisiert und/oder transportiert und/oder gelagert wird.

14. Verfahren nach einem der Ansprüche 1 bis 13, **dadurch gekennzeichnet, dass** das besiedelte, unbesiedelte oder teilweise besiedelte Substrat (B) durch Einleitung von Kühlflüssigkeit oder Gasen, wie insbesondere Stickstoff, zeitweise kryokonserviert wird.

15. Besiedlungsvorrichtung (10), bestehend aus
- einem Behälter (10a,b) und
- einer darin angeordneten Einspannvorrichtung (12) für ein zu besiedelndes Substrat (B), die so ausgebildet ist, dass unter Zusammenwirken mit dem eingespannten Substrat (B) innerhalb des Behälters (10a,b) wenigstens zwei im wesentlichen flüssigkeitstrennende oder einen kontrollierten Flüssigkeitstransfer ermöglichende Kammern auf entgegengesetzten Seiten des eingespannten Substrates (B) gebildet werden,
- und mit wenigstens je einem Flüssigkeitszu- und -ablauf (16a, 16a', 16b, 16b') für jede der gebildeten Kammern, **dadurch gekennzeichnet, dass** die Einspannvorrichtung (12) in einem herausnehmbaren Behältereinsatz (10b) angeordnet ist, so dass das Substrat (B) mit der Einspannvorrichtung (12) in den Behälter (10a) eingesetzt und aus diesem wieder entfernt werden kann.

16. Besiedlungsvorrichtung (10) nach Anspruch 15, **dadurch gekennzeichnet, dass** der Behälter (10a,b) rollbar ausgebildet ist.

17. Besiedlungsvorrichtung (10) nach Anspruch 15 oder 16, **dadurch gekennzeichnet, dass** die eine oder die mehreren Einspannvorrichtung(en) (12) im wesentlichen quer zu einer durch eine Rollbewegung des Behälters (10a,b) vorgegebene Vorzugsbewegungsrichtung eines darin befindlichen flüssigen Mediums angeordnet ist (sind.

18. Besiedlungsvorrichtung (10) nach einem der Ansprüche 15 bis 17, **dadurch gekennzeichnet, dass** die Einspannvorrichtung (12) in Bezug auf die Zu- und Abläufe (16a, 16a', 16b, 16b') so angeordnet ist, dass eine durch die Zu- und Abläufe vorgegebene Strömungsrichtung des flüssigen Mediums durch den Reaktor längs des eingespannten Substrates (B) verläuft.

19. Besiedlungsvorrichtung (10) nach einem der Ansprüche 15 bis 18, **dadurch gekennzeichnet, dass** die Einspannvorrichtung (12) aus wenigstens einer rahmenförmigen Halterung besteht.

20. Besiedlungsvorrichtung (10) nach einem der Ansprüche 15 bis 18, **dadurch gekennzeichnet, dass** die Einspannvorrichtung (12) wenigstens eine verzweigte Halterung für die Herstellung einer Y-Prothese aufweist.

21. Besiedlungsvorrichtung (10) nach einem der Ansprüche 15 bis 20,**dadurch gekennzeichnet, dass** die Einspannvorrichtung (12), vorzugsweise an einer Halterung, wenigstens einen weiteren Zu- und/oder Ablauf für das Einbringen von Medium, Zellen (3B) und/oder Material in wenigstens eine Innenkammer eines doppel- oder mehrwandigen Substrates (B) aufweist.

22. Besiedlungsvorrichtung (10) nach einem der Ansprüche 15 bis 21, **dadurch gekennzeichnet, dass** der rollbare Behälter (10a,b) zylinderförmig ist.

23. Besiedlungsvorrichtung (10) nach einem der Ansprüche 15 bis 22, **dadurch gekennzeichnet, dass** der rollbare Behälter (10a,b) kugelförmig oder kugelähnlich geformt ist.

24. Besiedlungsvorrichtung (10) nach einem der Ansprüche 15 bis 23, **dadurch gekennzeichnet, dass** wenigstens eine Kammer zusätzlich eine oder mehrere Gaszu- und -abführleitungen umfasst.

25. Besiedlungsvorrichtung (10) nach einem der Ansprüche 15 bis 24, **dadurch gekennzeichnet, dass** die Zu- und Abläufe und ggf. die Gaszu- und -abführleitungen über drehbare Kupplungen angeschlossen sind.

26. Besiedlungsvorrichtung (10) nach einem der Ansprüche 15 bis 25, **dadurch gekennzeichnet, dass** der Besiedlungsvorrichtungs-Behälter (10a,b) aus gasdurchlässigen Wänden besteht.

27. Besiedlungsvorrichtung (10) nach einem der Ansprüche 15 bis 26, **dadurch gekennzeichnet, dass** Einbauten im Innern des Behälters (10a,b) angeordnet sind, die das flüssige Medium, während es durch den Reaktor strömt, in gewünschter Weise leiten, wobei die Einbauten vorzugsweise an der Behälterinnenwand angeordnet sind.

28. Besiedlungsvorrichtung (10) nach einem der Ansprüche 15 bis 27, **dadurch gekennzeichnet, dass** an der Innenwand des Behälters (10a,b) mit Hilfe einer mikroporösen Trennwand (20) eine zusätzliche Kammer mit wenigstens einem Zu- und Ablauf (18a, 18b) gebildet wird.

29. Anordnung aus einer Besiedlungsvorrichtung (10) nach einem der Ansprüche 15 bis 28 und einer rollbar ausgebildeten Halterung (100).

30. Anordnung nach Anspruch 29, wobei die Rotationsachse der rollbaren Halterung (100) und eine Längsachse des in der Einspannvorrichtung (12) der Besiedlungsvorrichtung (10) eingespannten Substrats (B) in einem Winkel zueinander stehen.

31. Verwendung der Besiedlungsvorrichtung (10) nach einem der Ansprüche 15 bis 28 als Transport- und Lagerbehälter für ein besiedeltes Substrat (B).

## Claims

1. Method for the multilayered colonization of substrates (B) with biological cells (3B), in which, within a device (10), a substrate held therein and cells (3B) intended for the colonization are subjected to at least one colonization phase, during which the substrate (B) is rotated about at least one axis in or with the device (10), and the cells (3B) are repeatedly brought into contact with the substrate (B) during the rotational movement, **characterized in that**
- the substrate (B) is subjected, after or during one or more colonization phases, to at least one perfusion phase, during which the substrate (B) is perfused at least in areas of its outer or inner surface.

2. Method according to Claim 1, **characterized in that**, during a colonization phase, cells (3B) are supplied in a medium or are provided by brushing onto the substrate (B), introduction into the substrate (B) or coating of the substrate (B) with a composition comprising the cells (3B).

3. Method according to Claim 2, **characterized in that** factors or cofactors, in particular growth factors or chemotactic factors, are added to the cells (3B).

4. Method according to one of Claims 1 to 3, **characterized in that**, during a perfusion phase, the substrate (B) is perfused with a liquid medium, preferably a cell culture nutrient medium or blood.

5. Method according to one of Claims 1 to 4, **characterized in that** the rotation takes place simultaneously about at least two spatial axes in a superimposed rotational movement.

6. Method according to one of Claims 1 to 4, **characterized in that** the rotation takes place successively or alternately about at least two spatial axes.

7. Method according to one of Claims 1 to 6, **characterized in that** the colonization and perfusion phases take place alternately.

8. Method according to one of Claims 1 to 7, **characterized in that** the substrate (B) is subjected, after at least one colonization phase, to an additional rest phase during which no rotation and no perfusion take place.

9. Method according to one of Claims 1 to 8, **characterized in that** the method includes further phases with additional method steps for the treatment of the already colonized, partially colonized or still uncolonized substrate (B).

10. Method according to one of Claims 1 to 9, **characterized in that** the perfusion takes place in a pulsed manner.

11. Method according to one of Claims 1 to 10, **characterized in that** the substrate (B) is colonized with different cells (3B) in different areas.

12. Method according to one of Claims 1 to 10, **characterized in that** the substrate (B) is colonized with different cells (3B) successively or in different positions.

13. Method according to one of Claims 1 to 12, **characterized in that** the substrate (B) is additionally cellularized and/or sterilized and/or transported and/or stored in the device (10) serving for the treatment.

14. Method according to one of Claims 1 to 13, **characterized in that** the colonized, uncolonized or partially colonized substrate (B) is temporarily cryo-preserved by introduction of cooling liquid or gases, such as, in particular, nitrogen.

15. Colonization device (10), consisting of
- a container (10a,b) and
- a clamping device (12) arranged therein for a substrate (B) to be colonized, which device is designed in such a way that at least two chambers which essentially separate liquids or facilitate controlled liquid transfer are formed within the container (10a,b) on opposite sides of the clamped substrate (B) with interaction with the clamped substrate (B),
- and having at least one liquid feed and discharge (16a, 16a', 16b, 16b') for each of the chambers formed,
**characterized in that** the clamping device (12) is arranged in a removable container insert (10b), so that the substrate (B) with the clamping device (12) can be inserted into the container (10a) and removed therefrom again.

16. Colonization device (10) according to Claim 15, **characterized in that** the container (10a,b) is designed to be rollable.

17. Colonization device (10) according to Claim 15 or 16, **characterized in that** the one or more clamping device(s) (12) is (are) arranged essentially transversely to a preferential movement direction, defined by a rolling movement of the container (10a,b), of a liquid medium located therein.

18. Colonization device (10) according to one of Claims 15 to 17, **characterized in that** the clamping device (12) is arranged in such a way in relation to the feeds and discharges (16a, 16a', 16b, 16b') that a flow direction, defined by the feeds and discharges, of the liquid medium through the reactor runs along the clamped substrate (B).

19. Colonization device (10) according to one of Claims 15 to 18, **characterized in that** the clamping device (12) consists of at least one frame-shaped holder.

20. Colonization device (10) according to one of Claims 15 to 18, **characterized in that** the clamping device (12) has at least one branched holder for the production of a Y prosthesis.

21. Colonization device (10) according to one of Claims 15 to 20, **characterized in that** the clamping device (12) has, preferably on a holder, at least one further feed and/or discharge for the introduction of medium, cells (3B) and/or material into at least one internal chamber of a double- or multiwalled substrate (B).

22. Colonization device (10) according to one of Claims 15 to 21, **characterized in that** the rollable container (10a,b) is cylindrical.

23. Colonization device (10) according to one of Claims 15 to 22, **characterized in that** the rollable container (10a,b) has a spherical or spheroidal shape.

24. Colonization device (10) according to one of Claims 15 to 23, **characterized in that** at least one chamber additionally comprises one or more gas feed and discharge lines.

25. Colonization device (10) according to one of Claims 15 to 24, **characterized in that** the feeds and discharges and, where present, the gas feed and discharge lines are connected via rotatable couplings.

26. Colonization device (10) according to one of Claims 15 to 25, **characterized in that** the colonization device container (10a,b) consists of gas-permeable walls.

27. Colonization device (10) according to one of Claims 15 to 26, **characterized in that** internals which guide the liquid medium, while it flows through the reactor, in the desired manner are arranged in the interior of the container (10a,b), where the internals are preferably arranged on the inside wall of the container.

28. Colonization device (10) according to one of Claims 15 to 27, **characterized in that** an additional chamber having at least one feed and discharge (18a, 18b) is formed on the inside wall of the container (10a,b) with the aid of a microporous dividing wall (20).

29. Arrangement comprising a colonization device (10) according to one of Claims 15 to 28 and a holder (100) designed to be rollable.

30. Arrangement according to Claim 29, where the axis of rotation of the rollable holder (100) and a longitudinal axis of the substrate (B) clamped in the clamping device (12) of the colonization device (10) are at an angle to one another.

31. Use of the colonization device (10) according to one of Claims 15 to 28 as transport and storage container for a colonized substrate (B).

## Revendications

1. Procédé pour la colonisation multicouche de substrats (B) avec des cellules biologiques (3B), dans lequel,
à l'intérieur d'un dispositif (10), un substrat contenu dedans et des cellules (3B) destinées à la colonisation sont soumis à au moins une phase de colonisation pendant laquelle le substrat (B) est tourné autour d'au moins un axe dans ou avec le dispositif (10), et les cellules (3B) sont amenées de façon répétée en contact avec le substrat (B) pendant le mouvement de rotation,
**caractérisé en ce que**
- le substrat (B) est soumis, après ou pendant une ou plusieurs phases de colonisation, à au moins une phase de perfusion, pendant laquelle le substrat (B) est perfusé au moins dans des zones de sa surface externe ou interne.

2. Procédé selon la revendication 1, **caractérisé en ce que**, pendant une phase de colonisation, des cellules (3B) sont fournis dans un milieu ou par brossage sur le substrat (B), par introduction dans le substrat (B) ou par dépôt sur le substrat (B) d'une composition qui comprend les cellules (3B).

3. Procédé selon la revendication 2, **caractérisé en ce que** des facteurs ou des cofacteurs, en particulier des facteurs de croissance ou des facteurs chimio-tactiques, sont ajoutés aux cellules (3B).

4. Procédé selon l'une des revendications 1 à 3, **caractérisé en ce que**, pendant une phase de perfusion, le substrat (B) est perfusé avec un milieu liquide, de préférence un milieu de nourriture de culture de cellules ou du sang.

5. Procédé selon l'une des revendications 1 à 4, **caractérisé en ce que** la rotation est réalisée simultanément autour d'au moins deux axes spatiaux selon un mouvement de rotation superposé.

6. Procédé selon l'une des revendications 1 à 4, **caractérisé en ce que** la rotation est réalisée en succession ou en alternance autour d'au moins deux axes spatiaux.

7. Procédé selon l'une des revendications 1 à 6, **caractérisé en ce que** les phases de colonisation et de perfusion sont réalisées en alternance.

8. Procédé selon l'une des revendications 1 à 7, **caractérisé en ce que** le substrat (B) est soumis, après au moins une phase de colonisation, à une phase de repos additionnelle pendant laquelle aucune rotation et aucune perfusion ne sont réalisées.

9. Procédé selon l'une des revendications 1 à 8, **caractérisé en ce que** le procédé inclut en outre des phases avec des étapes de procédé additionnelles pour le traitement du substrat déjà colonisé, partiellement colonisé ou toujours non colonisé (B).

10. Procédé selon l'une des revendications 1 à 9, **caractérisé en ce que** la perfusion est réalisée d'une manière pulsée.

11. Procédé selon l'une des revendications 1 à 10, **caractérisé en ce que** le substrat (B) est colonisé avec différentes cellules (3B) dans différentes zones.

12. Procédé selon l'une des revendications 1 à 10, **caractérisé en ce que** le substrat (B) est colonisé avec différentes cellules (3B) en succession ou en des positions différentes.

13. Procédé selon l'une des revendications 1 à 12, **caractérisé en ce que** le substrat (B) est de façon additionnelle cellularisé et/ou stérilisé et/ou transporté et/ou stocké dans le dispositif (10) qui sert pour le traitement.

14. Procédé selon l'une des revendications 1 à 13, **caractérisé en ce que** le substrat colonisé, non colonisé ou partiellement colonisé (B) est cryo-conservé temporairement par introduction d'un liquide ou de gaz de refroidissement tel qu'en particulier de l'azote.

15. Dispositif de colonisation (10), constitué par :
- un conteneur (10a, 10b) ; et
- un dispositif de fixation (12) qui est agencé dedans pour un substrat (B) destiné à être colonisé, lequel dispositif est conçu de telle sorte qu'au moins deux chambres qui, de façon essentielle, séparent des liquides ou facilitent un transfert de liquide contrôlé soient formées à l'intérieur du conteneur (10a,b) sur des côtés opposés du substrat fixé (B) avec une interaction avec le substrat fixé (B),
- et comportant au moins une alimentation et décharge de liquide (16a, 16a', 16b, 16b') pour chacune des chambres formées,
**caractérisé en ce que** le dispositif de fixation (12) est agencé dans un insert de conteneur amovible (10b) de telle sorte que le substrat (B) avec le dispositif de fixation (12) puisse être inséré à l'intérieur du conteneur (10a) et puisse en être enlevé à nouveau.

16. Dispositif de colonisation (10) selon la revendication 15, **caractérisé en ce que** le conteneur (10a,b) est conçu de manière à pouvoir être roulé.

17. Dispositif de colonisation (10) selon la revendication 15 ou 16, **caractérisé en ce que** les un ou plusieurs dispositif(s) de fixation (12) est/sont agencé(s) essentiellement transversalement à une direction de déplacement préférentiel, comme défini par un mouvement de roulement du conteneur (10a,b), d'un milieu liquide localisé dedans.

18. Dispositif de colonisation (10) selon l'une des revendications 15 à 17, **caractérisé en ce que** le dispositif de fixation (12) est agencé de telle sorte que, par rapport aux alimentations et décharges (16a, 16a', 16b, 16b'), une direction de circulation, qui est définie par les alimentations et décharges, du milieu liquide au travers du réacteur courre le long du substrat fixé (B).

19. Dispositif de colonisation (10) selon l'une des revendications 15 à 18, **caractérisé en ce que** le dispositif de fixation (12) est constitué par au moins un support en forme de cadre.

20. Dispositif de colonisation (10) selon l'une des revendications 15 à 18, **caractérisé en ce que** le dispositif de fixation (12) comporte au moins un support en embranchement pour la production d'une prothèse Y.

21. Dispositif de colonisation (10) selon l'une des revendications 15 à 20, **caractérisé en ce que** le dispositif de fixation (12) comporte, de préférence sur un support, au moins une alimentation et/ou décharge supplémentaire(s) pour l'introduction d'un milieu, des cellules (3B) et/ou d'un matériau dans au moins une chambre interne d'un substrat à deux parois ou multiparoi (B).

22. Dispositif de colonisation (10) selon l'une des revendications 15 à 21, **caractérisé en ce que** le conteneur pouvant rouler (10a,b) est cylindrique.

23. Dispositif de colonisation (10) selon l'une des revendications 15 à 22, **caractérisé en ce que** le conteneur pouvant rouler (10a,b) présente une forme sphérique ou sphéroïdale.

24. Dispositif de colonisation (10) selon l'une des revendications 15 à 23, **caractérisé en ce qu'**au moins une chambre comprend de façon additionnelle une ou plusieurs lignes d'alimentation et de décharge de gaz.

25. Dispositif de colonisation (10) selon l'une des revendications 15 à 24, **caractérisé en ce que** les alimentations et décharges et, lorsqu'elles sont présentes, les lignes d'alimentation et de décharge de gaz, sont connectées via des couplages pouvant tourner.

26. Dispositif de colonisation (10) selon l'une des revendications 15 à 25, **caractérisé en ce que** le conteneur de dispositif de colonisation (10a,b) est constitué par des parois perméables aux gaz.

27. Dispositif de colonisation (10) selon l'une des revendications 15 à 26, **caractérisé en ce que** des parties internes qui guident le milieu liquide, tandis qu'il s'écoule au travers du réacteur, selon la manière souhaitée sont agencées à l'intérieur du conteneur (10a,b), où les parties internes sont de préférence agencées sur la paroi interne du conteneur.

28. Dispositif de colonisation (10) selon l'une des revendications 15 à 27, **caractérisé en ce qu'**une chambre additionnelle comportant au moins une alimentation et décharge (18a, 18b) est formée sur la paroi interne du conteneur (10a,b) à l'aide d'une paroi de division microporeuse (20).

29. Agencement comprenant un dispositif de colonisation (10) selon l'une des revendications 15 à 28 et un support (100) conçu de manière à pouvoir être roulé.

30. Agencement selon la revendication 29, où l'axe de rotation du support (100) pouvant rouler et un axe longitudinal du substrat (B) qui est fixé dans le dispositif de fixation (12) du dispositif de colonisation (10) sont selon un certain angle l'un par rapport à l'autre.

31. Utilisation du dispositif de colonisation (10) selon l'une des revendications 15 à 28 en tant que conteneur de transport et de stockage pour un substrat colonisé (B).
